**Europäisches Patentamt**

**European Patent Office**

**Office européen des brevets**

(19)

(11) Veröffentlichungsnummer : **0 438 665 B1**

## (12) EUROPÄISCHE PATENTSCHRIFT

(45) Veröffentlichungstag der Patentschrift :
**01.09.93 Patentblatt 93/35**

(51) Int. Cl.$^5$ : **C07C 315/04, C07C 317/22**

(21) Anmeldenummer : **90122507.8**

(22) Anmeldetag : **26.11.90**

(54) **Verfahren zur Herstellung von 4,4'-Bis-(4-aminophenoxy)-diphenylsulfon.**

(30) Priorität : **23.01.90 DE 4001821**

(43) Veröffentlichungstag der Anmeldung :
**31.07.91 Patentblatt 91/31**

(45) Bekanntmachung des Hinweises auf die
Patenterteilung :
**01.09.93 Patentblatt 93/35**

(84) Benannte Vertragsstaaten :
**AT BE CH DE FR GB IT LI NL**

(56) Entgegenhaltungen :
**EP-A- 0 192 480**
**DE-A- 1 909 520**
**DE-A- 2 315 607**
**US-A- 3 988 374**

(56) Entgegenhaltungen :
**JOURNAL OF POLYMER SCIENCE: POLYMER CHEMISTRY EDITION, Band 12, 1974, Seiten 565-573, John Wiley & Sons, Inc., New York, NY, US; J.H. KAWAKAMI et al.: "High temperature polymers. I. - Sulfone ether diamines as intermediates for tractable high temperature polymers"**
**CHEMICAL ABSTRACTS, Band 73, 1970, Seite 287, Zusammenfassung Nr. 45124s, Columbus, Ohio, US; & JP-A-70 15 020**

(73) Patentinhaber : **HÜLS AKTIENGESELLSCHAFT**
**D-45764 Marl (DE)**

(72) Erfinder : **Bartmann, Martin, Dr.**
**Elper Weg 64**
**W-4350 Recklinghausen (DE)**
Erfinder : **Finke, Jürgen, Dr.**
**Münsterlandstrasse 22**
**W-4370 Marl (DE)**
Erfinder : **Poll, Günter, Dr.**
**Venusweg 9**
**W-4370 Marl (DE)**
Erfinder : **Ribbing, Wilfried, Dr.**
**Heesternweg 20**
**W-4270 Dorsten 12 (DE)**

## Beschreibung

Die Erfindung betrifft ein neues Verfahren zur Herstellung von reinem 4,4'-Bis-(4-aminophenoxy)-diphenylsulfon aus 4-Aminophenol, Alkalihydroxid und 4,4'-Dihalogendiphenylsulfon in einem organischen Lösemittel.

4,4'-Bis-(4-aminophenoxy)-diphenylsulfon (BAPS) ist ein wichtiges Monomer zur Herstellung von hochschmelzenden Polyamiden, Polyimiden und Polyamidimiden. Es ist darüber hinaus auch als Härter von Epoxiden geeignet.

BAPS wird nach DE-A-19 09 520 aus einem Alkalisalz des p-Aminophenols und einem 4,4'-Dihalogendiphenylsulfon in einem polaren, aprotischen Lösemittel, das auch ein N-alkyliertes Säureamid sein kann, bei 50 bis 150 °C hergestellt. Dabei werden p-Aminophenol und Alkalilauge in äquivalenten Mengen und die Dihalogenverbindung in geringem Unterschuß, bezogen auf die stöchiometrisch erforderliche Menge des p-Aminophenols, eingesetzt. Im Beispiel liegt das Molverhältnis von p-Aminophenol zu 4,4'-Dichlordiphenylsulfon bei 2,063. Die Reaktion wird in Dimethylsulfoxid bei 100 °C ausgeführt. Das Produkt wird mit Wasser gefällt, gelöst, mit Aktivkohle behandelt und erneut gefällt. Es hat dann einen Schmelzpunkt von 191 bis 192 °C, der durch Umkristallisation auf 193 bis 194 °C erhöht werden kann.

Nach DE-A-23 15 607 wird BAPS aus p-Aminophenol, Natronlauge und 4,4'-Dichlordiphenylsulfon hergestellt, wobei stöchiometrische Mengen der Ausgangsverbindungen eingesetzt werden. Die Reaktion wird in Dimethylsulfoxid bei 160 °C durchgeführt. Nach zweifacher Fällung wird ein Produkt mit einem Schmelzpunkt von 177 bis 187 °C erhalten. Nach Umkristallisation liegt der Schmelzpunkt bei 188 bis 191 °C.

Auch nach Kawakami et al. (Journal of Polymer Science, Polymer Chemistry Edition, Vol. 12 (1974), 565 - 73) wird BAPS aus p-Aminophenol, Natriumhydroxid und 4,4'-Dichlordiphenylsulfon in Dimethylsulfoxid hergestellt. Dabei wird, bezogen auf Natriumhydroxid, ein 1- bis 2%iger molarer Überschuß an p-Aminophenol vorgeschlagen. Vor einem Natriumhydroxid-Überschuß wird gewarnt, da dadurch N-Brücken aufgebaut werden könnten. In einem Ausführungsbeispiel beträgt das Molverhältnis von Natriumhydroxid zu p-Aminophenol 0,99 und das von p-Aminophenol zu 4,4'-Dichlordiphenylsulfon 2,10. Man erhält dabei ein Produkt mit einem Schmelzpunkt von 189 bis 191 °C. Nach Umkristallisation liegt der Schmelzpunkt bei 191 bis 192 °C.

Für Polykondensationszwecke wird ein möglichst sauberes BAPS mit einem möglichst hohen Schmelzpunkt benötigt. Dabei ist BAPS mit einem Schmelzpunkt unter 191 °C noch ungeeignet, wohingegen ein Produkt mit einem Schmelzpunkt von 191 bis 193 °C nur zu minderwertigen Polykondensaten mit dunkler Farbe und schlechten mechanischen Eigenschaften führt. Die bekannten Verfahren liefern demnach nur nach mehreren Reinigungsschritten und nach Umkristallisation ein für Polykondensationen gut oder bedingt einsatzbares BAPS.

Aufgabe der vorliegenden Erfindung war es, ein vereinfachtes Verfahren zur Herstellung von BAPS hoher Reinheit zu entwickeln. Dabei sollte nach Möglichkeit bereits nach beendeter Reaktion ein Produkt anfallen, das auch ohne Umkristallisation für Polykondensationszwecke gut geeignet ist.

Diese Aufgabe wird nach Anspruch 1 durch 3 Maßnahmen gelöst:

1. 1,0 bis 1,1 Mole Alkalihydroxid pro Mol 4-Aminophenol,
2. 2,005 bis 2,05 Mole 4-Aminophenol pro Mol 4,4'-Dihalogendiphenylsulfon,
3. Dialkylamid als Lösemittel.

Dabei wird Alkalihydroxid vorzugsweise mit 1,01 bis 1,05 Molen pro Mol 4-Aminophenol in einem kleinen Überschuß angewandt. Als Alkalihydroxid wird bevorzugt Natrium- oder Kaliumhydroxid eingesetzt. Das Alkalihydroxid wird im allgemeinen als wäßrige Lauge zugesetzt, wobei man 10- bis 60%ige Lösungen bevorzugt. Nach Bildung des Phenolats wird das Wasser wieder entfernt. Dieses kann mit Vorteilen mit Hilfe eines organischen Lösemittels durch eine azeotrope Destillation erfolgen.

Das Molverhältnis von 4-Aminophenol zu 4,4'-Dihalogendiphenylsulfon beträgt vorzugsweise 2,01 bis 2,04. Dabei kommen als Dihalogenverbindung vor allem die Dichlor- und die Dibromverbindung in Betracht. 4,4'-Dichlordiphenylsulfon wird bevorzugt eingesetzt.

Für die Umsetzung des Alkaliphenolats mit dem 4,4'-Dihalogendiphenylsulfon sind Dialkylamide als Lösemittel geeignet. Beispiele dafür sind Dimethylformamid, Dimethylacetamid, Dimethylbenzamid, N-Methylpyrrolidon, N-Ethylpyrrolidon, N-Methylpiperidon, N-Methylcaprolactam und N-Methyllaurinlactam. Dabei wird N-Methylpyrrolidon als Lösemittel bevorzugt.

Die Umsetzung wird vorzugsweise in einer 20- bis 85%igen Lösung, bezogen auf das Phenolat und die Dihalogenverbindung, durchgeführt. Die bevorzugte Reaktionstemperatur liegt zwischen 150 und 180 °C, die Reaktion ist im allgemeinen nach 1 bis 20 Stunden beendet. Die Reaktionszeit liegt meist bei 2 bis 10 Stunden.

Die Umsetzung von Alkaliphenolat und Dihalogenverbindung kann auch in Gegenwart von Reduktionsmitteln wie Natriumhypophosphit oder Natriumdithionit durchgeführt werden. Dadurch kann bei nicht quantitativem Ausschluß von Sauerstoff eine Bildung von Chinonen und eine Dunkelfärbung des Produkts vermieden

2

werden. Man setzt bis zu 5 Mol-% Reduktionsmittel, bezogen auf die Dihalogenverbindung, ein.

BAPS wird aus der Reaktionslösung mit Hilfe eines Fällungsmittels gefällt. Dazu sind Mischungen aus Alkohol und Wasser, aus Kohlenwasserstoff und Wasser oder aus Kohlenwasserstoff, Alkohol und Wasser geeignet. Als Fällungsmittel werden vorzugsweise Alkohol/Wasser-Mischungen verwendet. Geeignete sind dabei niedere, mit Wasser mischbare Alkohole, wie Methanol, Ethanol, Isopropanol und n-Propanol. Man kann auch mit Wasser nicht mischbare Alkohole, wie z. B. Butanol, Hexanol oder Octanol, einsetzen. Die Mischungen können 10 bis 90 % Alkohol enthalten.

Nach dem erfindungsgemäßen Verfahren wird ein BAPS hergestellt, das auch ohne Umkristallisation einen Schmelzpunkt von über 193 °C aufweist. Mit einem überraschend geringen Reinigungsaufwand wird sogar ein BAPS erhalten, das mindestens 99,5%ig ist und dabei einen Schmelzpunkt von mindestens 195 °C aufweist. Es kann direkt zur Polykondensation von hochmolekularen, hochschmelzenden Polymeren eingesetzt werden.

Bei der Durchführung des erfindungsgemäßen Verfahrens wird allgemein wie folgt vorgegangen:

4-Aminophenol wird in einem mit Alkalilauge mischbaren Lösemittelgemisch aus einem Dialkylamid und beispielsweise einem Kohlenwasserstoff gelöst. Dabei sollte mindestens ein Lösemittel mit Wasser ein Azeotrop bilden können. Geeignete Kohlenwasserstoffe, die mit Wasser Azeotrope bilden, sind Benzol, Toluol, Hexan, Heptan, Cyclohexan und andere. Die Lösung wird durch Spülen mit einem Inertgas, wie Stickstoff oder Argon, von Sauerstoff befreit. Anschließend kann man ein Reduktionsmittel zusetzen, um gegebenenfalls noch vorhandene Sauerstoffspuren zu entfernen.

Zu der Lösung des 4-Aminophenols gibt man die wäßrige Alkalilauge bei einer Temperatur zwischen Raumtemperatur und dem Siedepunkt des eingesetzten Kohlenwasserstoffs. Wenn die Bildung des Phenolats abgeschlossen ist, wird das Wasser durch azeotrope Destillation entfernt.

Zu der verbleibenden Lösung des Aminophenolats in dem Dialkylamid und dem Kohlenwasserstoff wird dann eine Lösung des 4,4'-Dihalogendiphenylsulfons in einem Dialkylamid gegeben, wobei man vorzugsweise in beiden Lösungen das gleiche Dialkylamid verwendet. Die Reaktionsmischung wird auf die Reaktionstemperatur erhitzt, wobei der Kohlenwasserstoff abdestilliert.

Nach beendeter Reaktion kühlt man die Reaktionsmischung auf unter 100 °C ab und setzt das Fällungsmittel zu. Das ausgefällte Produkt wird abfiltriert, mit Wasser, Alkohol oder einer Wasser/Alkohol-Mischung gewaschen und getrocknet.

In den folgenden Beispielen werden die erfindungsgemäßen Beispiele durch Zahlen und die Vergleichsbeispiele durch Buchstaben gekennzeichnet.

Die nachfolgend angegebenen Schmelzpunkte von BAPS wurden nach der DSC-Methode bei einer Aufheizgeschwindigkeit von 10 °C/min ermittelt.

Die Reinheit des Diamins wurde dünnschichtchromatographisch auf Kieselgel mit Chloroform bestimmt. Die quantitative Auswertung erfolgte UV-spektroskopisch.

## Beispiel 1

Zu einer mit Stickstoff gesättigten Lösung von 109,5 g (1,0 mol) 4-Aminophenol in 100 ml Toluol und 100 ml N-Methylpyrrolidon werden bei Raumtemperatur 42 g (1,05 mol) NaOH (als 25%ige wäßrige Lösung) zugegeben. Diese Mischung wird auf eine Innentemperatur von ca. 110 °C erhitzt, wobei die Destillation des Toluol/Wasser-Azeotrops einsetzt. Mit Hilfe eines Wasserabscheiders wird das Wasser abgetrennt und das Toluol zurückgeführt. Nachdem das Wasser vollständig abdestilliert ist, wird der Ansatz auf 60 °C abgekühlt. Bei dieser Temperatur wird eine Lösung von 140,9 g (0,49 mol) 4,4'-Dichlordiphenylsulfon in 100 ml N-Methylpyrrolidon zugegeben. Anschließend wird die Innentemperatur kontinuierlich auf 170 °C gesteigert. Zwischenzeitlich wird bei ca. 110 °C Innentemperatur das eingesetzte Toluol abdestilliert. Die Temperatur wird 5 Stunden bei 170 °C gehalten und danach auf 70 °C gesenkt. Bei dieser Temperatur werden langsam 1,5 l einer Wasser/Methanol-Mischung (1 : 1) zugegeben, wobei das entstandene 4,4'-Bis-(4-aminophenoxy)-diphenylsulfon ausfällt. Der Ansatz wird auf Raumtemperatur abgekühlt. Das ausgefallene Produkt wird abfiltriert, mit Wasser gewaschen und getrocknet.

Ausbeute : 203,4 g = 96 % d. Th.
$T_m$ : 195,1 °C
Reinheit : 99,6 %

## Beispiele 2 bis 7 und Vergleichsbeispiele A und B

Es wird wie in Beispiel 1 verfahren. Die Mengenverhältnisse sowie die Bedingungen und Ergebnisse der Reaktionen sind in Tabelle 1 zusammengestellt.

Tabelle 1

| Bsp. | Molverhältnis DCDPS[1] : AP[2] : Base | Base | $Na_2S_2O_4$ [Mol-%][3] | Reaktion Temp.[°C] | Zeit [h] | Fällungsmittel | Ausbeute [%] | $T_m$ [°C] | Reinheit [%] |
|---|---|---|---|---|---|---|---|---|---|
| 1 | 1,0 : 2,041 : 2,143 | NaOH | - | 170 | 5 | $H_2O$/MeOH[4] | 96 | 195,1 | 99,6 |
| 2 | 1,0 : 2,009 : 2,01 | NaOH | - | 140 | 7 | $H_2O$/MeOH | 91 | 195,0 | 99,5 |
| 3 | 1,0 : 2,015 : 2,075 | NaOH | 1,0 | 140 | 10 | $H_2O$/EtOH[5] | 89 | 195,1 | 99,6 |
| 4 | 1,0 : 2,023 : 2,065 | KOH | 2,0 | 180 | 4 | $H_2O$/EtOH | 91 | 194,5 | 99,3 |
| 5 | 1,0 : 2,030 : 2,04 | KOH | - | 150 | 5 | $H_2O$/i-PrOH[6] | 93 | 195,8 | 99,9 |
| 6 | 1,0 : 2,038 : 2,10 | NaOH | - | 160 | 5 | $H_2O$/i-PrOH | 94 | 195,5 | 99,7 |
| 7 | 1,0 : 2,049 : 2,10 | NaOH | - | 170 | 5 | $H_2O$/MeOH | 96 | 195,1 | 99,6 |
| A | 1,0 : 2,0 : 2,0 | NaOH | - | 170 | 5 | $H_2O$/MeOH | 88 | 191,7 | 97,8 |
| B | 1,0 : 2,1 : 2,05 | NaOH | - | 170 | 5 | $H_2O$/MeOH | 86 | 191,2 | 97,5 |

1) 4,4'-Dichlordiphenylsulfon   2) 4-Aminophenol   3) bezogen auf DCDPS
4) Methanol   5) Ethanol   6) Isopropanol

**Patentansprüche**

1. Verfahren zur Herstellung von 4,4'-Bis-(4-aminophenoxy)-diphenylsulfon mit hoher Reinheit, bei dem man

4

4-Aminophenol mit einem Alkalihydroxid in das entsprechende Alkaliphenolat überführt und dieses dann mit einem 4,4'-Dihalogendiphenylsulfon in einem organischen Lösemittel bei 120 bis 200 °C umsetzt, worauf das Produkt mit Hilfe eines Fällungsmittels ausgefällt und dann abgetrennt wird, dadurch gekennzeichnet, daß man

- 1,0 bis 1,1 Mole Alkalihydroxid pro Mol 4-Aminophenol,
- 2,005 bis 2,05 Mole 4-Aminophenol pro Mol 4,4'-Dihalogendiphenylsulfon und
- als organisches Lösemittel ein Dialkylamid einsetzt.

2. Verfahren nach Anspruch 1, dadurch gekennzeichnet, daß man 1,01 bis 1,05 Mole Alkalihydroxid pro Mol 4-Aminophenol verwendet.

3. Verfahren nach Anspruch 1, dadurch gekennzeichnet, daß man 2,01 bis 2,04 Mole 4-Aminophenol pro Mol 4,4'-Dihalogendiphenylsulfon einsetzt.

4. Verfahren nach Anspruch 1, dadurch gekennzeichnet, daß man als Lösemittel N-Methylpyrrolidon verwendet.

5. Verfahren nach Anspruch 1, dadurch gekennzeichnet, daß man die Umsetzung in Gegenwart von bis zu 5 Mol-% Reduktionsmittel, bezogen auf 4,4'-Dihalogendiphenylsulfon, durchführt.

6. Verfahren nach Anspruch 1, dadurch gekennzeichnet, daß man als Alkalihydroxid Natrium- und/oder Kaliumhydroxid einsetzt.

7. Verfahren nach Anspruch 1, dadurch gekennzeichnet, daß 4,4'-Dichlordiphenylsulfon verwendet wird.

8. Verfahren nach Anspruch 1, dadurch gekennzeichnet, daß man die Umsetzung von Alkaliphenolat und 4,4'-Dihalogendiphenylsulfon bei einer Temperatur zwischen 150 und 180 °C durchführt.

9. Verfahren nach Anspruch 1, dadurch gekennzeichnet, daß man das Produkt mit Hilfe einer Alkohol/Wasser-Mischung ausfällt.

## Claims

1. A process for the preparation of 4,4'-bis-(4-aminophenoxy)diphenyl sulphone having high purity, in which 4-aminophenol is converted, by an alkali metal hydroxide, into the corresponding alkali metal phenolate and this is then reacted with a 4,4'-dihalodiphenyl sulphone in an organic solvent at 120 to 200°C, whereupon the product is precipitated out with the aid of a precipitant and then separated off, characterised in that

- from 1.0 to 1.1 mol of alkali metal hydroxide are used per mol of 4-aminophenol,
- from 2.005 to 2.05 mol of 4-aminophenol are used per mol of 4,4'-dihalodiphenyl sulphone and
- the organic solvent used is a dialkylamide.

2. A process according to claim 1, characterised in that from 1.01 to 1.05 mol of alkali metal hydroxide are used per mol of 4-aminophenol.

3. A process according to claim 1, characterised in that from 2.01 to 2.04 mol of 4-aminophenol are used per mol of 4,4'-dihalodiphenyl sulphone.

4. A process according to claim 1, characterised in that the solvent used is N-methylpyrrolidone.

5. A process according to claim 1, characterised in that the reaction is carried out in the presence of up to 5 mol % of reducing agent, based on 4,4'-dihalodiphenyl sulphone.

6. A process according to claim 1, characterised in that the alkali metal hydroxide used is sodium hydroxide and/or potassium hydroxide.

7. A process according to claim 1, characterised in that 4,4'-dichlorodiphenyl sulphone is used.

8. A process according to claim 1, characterised in that the reaction of alkali metal phenolate and 4,4'-dihalodiphenyl sulphone is carried out at a temperature of from 150 to 180°C.

9. A process according to claim 1, characterised in that the product is precipitated out with the aid of an alcohol/water mixture.


**Revendications**

1. Procédé de préparation de 4,4'-bis-(4-aminophénoxy)-diphényl-sulfone de grande pureté, dans lequel on transforme du 4-amino-phénol avec un hydroxyde alcalin en le phénolate alcalin correspondant et fait ensuite réagir celui-ci sur une 4,4'-dihalogéno-diphényl-sulfone, dans un solvant organique à une température de 120 à 200°C, après quoi on précipite le produit à l'aide d'un agent de précipitation et le sépare ensuite,
   caractérisé par le fait :
   - que l'on utilise de 1,0 à 1,1 mole d'hydroxyde alcalin par mole de 4-amino-phénol,
   - que l'on utilise de 2,005 à 2,05 moles de 4-amino-phénol par mole de 4,4'-dihalogéno-diphényl-sulfone, et
   - que l'on utilise une dialkylamide en tant que solvant organique.

2. Procédé selon la revendication 1,
   caractérisé par le fait que l'on utilise de 1,01 à 1,05 mole d'hydroxyde alcalin par mole de 4-amino-phénol.

3. Procédé selon la revendication 1,
   caractérisé par le fait que l'on utilise de 2,01 à 2,04 moles de 4-amino-phénol par mole de 4,4'-dihalogéno-diphényl-sulfone.

4. Procédé selon la revendication 1,
   caractérisé par le fait que l'on utilise comme solvant la N-méthyl-pyrrolidone.

5. Procédé selon la revendication 1,
   caractérisé par le fait que l'on effectue la réaction en présence d'une quantité d'agent réducteur allant jusqu'à 5 mole-%, relativement à la 4,4'-dihalogéno-diphényl-sulfone.

6. Procédé selon la revendication 1,
   caractérisé par le fait que l'on utilise, comme hydroxyde alcalin, l'hydroxyde de sodium et/ou l'hydroxyde de potassium.

7. Procédé selon la revendication 1,
   caractérisé par le fait qu'on utilise la 4,4'-dichlorodiphényl-sulfone.

8. Procédé selon la revendication 1,
   caractérisé par le fait que l'on effectue, à une température comprise entre 150 et 180°C, la réaction du phénolate alcalin et de la 4,4'-dihalogéno-diphényl-sulfone.

9. Procédé selon la revendication 1,
   caractérisé par le fait que l'on fait précipiter le produit à l'aide d'un mélange d'alcool et d'eau.